(19) Europäisches Patentamt
European Patent Office
Office européen des brevets

(11) Publication number: 0 186 428
B1

(12) EUROPEAN PATENT SPECIFICATION

(45) Date of publication of patent specification: 17.11.88

(51) Int. Cl.$^4$: C 07 C 143/12, C 08 F 2/26

(21) Application number: 85309226.0

(22) Date of filing: 18.12.85

# (54) Copolymerizable ethylenically unsaturated surfactants and their use in polymerization processes.

(30) Priority: 20.12.84 US 683973

(43) Date of publication of application:
02.07.86 Bulletin 86/27

(45) Publication of the grant of the patent:
17.11.88 Bulletin 88/46

(84) Designated Contracting States:
AT BE CH DE FR GB IT LI LU NL SE

(56) References cited:
DE-A-1 668 651
DE-A-3 149 201
DE-A-3 207 222
DE-A-3 317 336
US-A-4 049 608
US-A-4 442 043

(73) Proprietor: Rohm and Haas Company
Independence Mall West
Philadelphia, Pennsylvania 19105 (US)

(72) Inventor: Beckley, Ronald Scott
513 Kleman Road
Gilbertsville, Pa. 19525 (US)
Inventor: Randow, Rodney Lamar
753 Hoover Road
Blue Bell, Pa. 19422 (US)
Inventor: Swift, Graham
901 Cross Lane
Blue Bell, Pa. 19422 (US)

(74) Representative: Angell, David Whilton et al
ROHM AND HAAS (UK) LTD. European
Operations Patent Department Lennig House 2
Mason's Avenue
Croydon CR9 3NB (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 186 428 B1

Courier Press, Leamington Spa, England.

## Description

This invention is concerned with copolymerizable ethylenically unsaturated surfactants and their use in polymerization processes to control polymer particle size and stability, particularly their use in emulsion polymerization to control latex particle size and stability.

While surfactants are widely used in emulsion polymerizations, for most subsequent applications of the resulting latex, for example in coatings, adhesives and binders, their presence has been found to be detrimental. Water resistance, adhesion, corrosion resistance, and/or block resistance of films formed from such latexes may be adversely affected, and frequently such films exhibit an increased tendency to foam. Also, the stability of formulations comprising such latexes may be adversely affected.

The use of ultrafine latexes has been an important advantage in latex coating technology and often can impart the important properties of chalk adhesion, better film formation and water resistance. The conventional method of making such ultrafine latexes, however, requires large amounts of surfactant because of the large latex particle surface area. Therefore, the improvement due to ultrafine latexes can be transitory and lost on aging, formulation, heating, or a combination of these. Presumably, the surfactant equilibrates with the surfaces of all the materials in the formulation.

Various polymerizable surfactants have been described in the literature and are available commercially. One such series of surfactant is described in U.S. Patent 4,049,608 to Steckler et al. However, the Steckler et al surfactants suffer from the disadvantage of only having two allylic protons per molecule. Some other commercial surfactants have relatively reactive graft sites, such as allylesters and maleates. The problem with most of the prior polymerizable surfactants is that virtually all of them have the reactive group in the wrong end of the surfactant molecule.

The compounds of the present invention are capable of acting as polymerizable surfactants in polymerization processes, for example the emulsion polymerization of one or more unsaturated monomers, enabling efficient control of latex particle size, and also enabling the production of latexes having low foam tendencies, low water sensitivity in dried film formed from such latexes, improved block resistance, and improved latex stability.

According to the present invention there are provided compounds of the formula:—

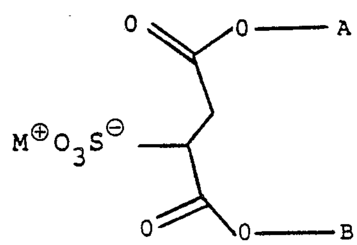

wherein M is a cation which is alkali metal, ammonium, amino or hydrogen; and each of A and B is a group which, together with the carboxyl group to which it is attached, forms an ester, at least one of A and B being a group having the formula:—

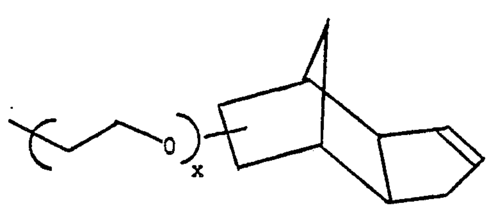

wherein x is from 0 to 5.

In one embodiment of the invention one of A and B is an alkyl group of 2 to 8 carbon atoms, preferably octyl.

In a preferred embodiment of the invention one of A and B is a group having the formula:—

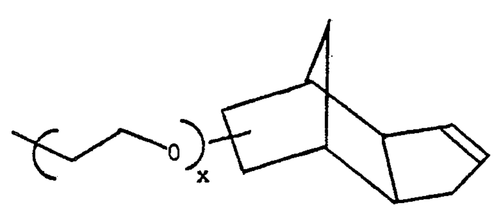

wherein x is from 0 to 5, and the other of A and B is a group having the formula:—

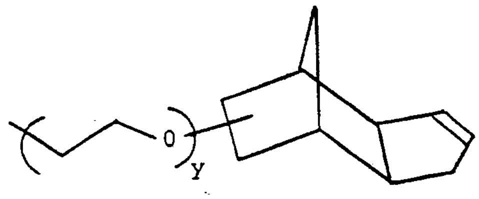

wherein y is from 0 to 5.

The compounds of the invention can be prepared by reaction of dicyclopentadiene and, depending on the desired value of x and y, one or more of ethylene glycol, diethylene glycol, triethylene glycol, tetraethylene glycol and pentaethylene glycol. The resultant tricyclo $[5.2.1.0^{2,6}]$dec-3-en-(8 or 9 oxyethyl)$_n$ alcohol (wherein n is from 0 to 5) optionally with another alcohol when the mixed esters are desired, is then reacted with maleic anhydride to form the disubstituted maleate and with bisulfite ion to form the compounds of the invention. Preferred methods are when n = 0, when dicyclopentenyl formate is transesterified with dialkyl maleate or fumarate and/or when none of the other optional alcohol is used.

The compounds of the invention are capable of being used as graftable and/or copolymerizable surfactants with unsaturated comonomers. The most frequently used comonomers are acrylates, methacrylates, styrene, substituted styrenes, acrylonitrile, methacrylonitrile, vinyl alkanoates, and butadienes.

In polymerization processes, for example emulsion copolymerization, the surfactant compound of the invention may, for example, be present in an amount of 0.1 to 5%, preferably, 0.5 to 3%, and more preferably 1 to 3%, by weight based on the weight of monomer(s).

The weight average diameter of the particles of a latex made by the process of the invention is preferably 40 to 200 nanometers and more preferably in the ultrafine range of 50 to 110 nanometers.

Latexes made by the process of the invention may be used in normal latex applications, for example, for paints, coatings, adhesives, roof mastics, caulks or non-woven binders.

An advantage of the compounds of the present invention is that they enable the production of latexes having improved stability because the surfactant cannot be readily extracted by other materials in formulations comprising such latexes. Further, the resultant latexes formed using the compounds of the invention conveniently have improved low foaming tendencies, lower water sensitivity in dried films formed therefrom, and improved block resistance.

The present invention will now be further illustrated by way of the following Examples which are for illustrative purposes only and are not to be construed as imposing any limitation on the scope of the invention.

## Example 1

A dried, 2 litre, 4-neck, round-bottom flask fitted with thermometer, addition funnel, stirrer and condenser was charged with 392 g (4 mol) of maleic anhydride (freshly pulverized briquette). With the starting materials at room temperature and with stirring, about one fourth of a 777.2 g (4 mol) charge of tricyclo$[5.2.1.0^{2,6}]$ dec-3-en(8 or 9) oxyethyl alcohol (*1*) was added to the flask. The remainder of *1* was then added to the flask over a period of about 1 hr and the resulting mixture was then heated to 50°C. After heating at about 50°C for 19 h, the conversion of alcohol and anhydride to ester was found to be about 87% of the theoretical conversion. p-methoxy phenol (MEHQ) (0.12 g) was added and the reaction mixture was heated at about 70°—77°C for an additional 6 h. Final conversion was 91%. The reaction mixture was monitored by nmr for conversion. The adduct has a singlet at delta 6.85 ppm (2 h, HC=CH) and unreacted maleic anhydride has a singlet at delta 7.3 ppm (2 h) ppm. The product produced was tricyclo$[5.2.1.0^{2,6}]$dec-3-en-(8 or 9) oxyethyl maleate (*2*).

## Example 2

A 500-ml, 3-necked, round-bottom flask fitted with Dean-Stark trap, condenser, stirrer and thermometer was charged with 146 g (0.5 mol) of *2*, 97 g (0.5 mol) of *1* and 125 g toluene to form a solution. After thorough mixing of the solution 1.5 g of concentrated sulfuric acid was added. The reaction mixture was then heated to reflux (110—132°C) and 8.1 ml (0.45 mol) of water was separated in the Dean-Stark trap. The resultant reaction mixture was then cooled and extracted successively with water, 10% sodium carbonate solution and brine. Emulsion formed readily and long times were necessary to get adequate separation. The washed organic layer was diluted with toluene and dried over anhydrous magnesium sulfate. The toluene was removed from the product by evaporation at temperatures up to 100°C on a rotary evaporator. 1.7% by weight of the product was retained toluene. The nmr spectra of the product was consistent with the desired diester, which was di-(tricyclo$[5.2.1.0^{2,6}]$ dec-3-en(8 or 9) oxyethyl) maleate (*3*).

## Example 3

A 500-ml, 3-necked, round-bottom flask fitted with a stirrer, thermometer, and condenser was charged with 187.2 g (0.4 mol) of *3*, 43.7 g (0.42 mol) sodium metabisulfite, and 40 g water. An outlet hose from the condenser terminated in about 3.81 cm (1.5") silicon oil to create a slight positive pressure. The reaction mixture was heated to about 90°C and within about 2 h had exothermed slightly to 100°C and then cooled to

95°C. During this time the reaction mixture became homogeneous, clear and soluble in water. About 25 mg of MEHQ was added to the product of the reaction. The resulting product, sodium di-(tricyclo[5.2.1.$0^{2,6}$]dec-3-en(8 or 9) oxyethyl) sulfosuccinate (*4*), was clear, homogeneous, dark brown and had a slight odour of sulfur dioxide.

Example 4

By modifying Examples 2 and 3, starting with the monoester *2* and various alcohols, a series of mixed sulfosuccinate surfactants, each bearing an average of one tricyclodecenyl moiety, were prepared. Since the step to prepare the diester is strong acid catalyzed, the actual product is expected to be a mixture of the all possible diester maleates, including the esters in which both ester groups are the cyclic groups (Fumarates might also be formed in any of the reactions, but after addition of the bisulfite it also yields the sulfosuccinate). In this way, mixed diester sulfosuccinates with one of the ester groups being ethyl, butyl, octyl or Carbitol, e.g. butyl Carbitol, were prepared.

Comparative Example A

Sodium di(tricyclo[5.2.1.$0^{2,6}$]-decan-8-oxyethyl) sulfosuccinate (*6*), which is a saturated analog of compound *4*, was prepared as follows:

A pressure vessel was charged with 58 g (0.3 mol) of alcohol *1*, 300 g ethyl alcohol (denatured 3A) and platinum dioxide (small spatula). The mixture was hydrogenated on a Parr apparatus with 19.96—9.07 Kg (44—20 lb) hydrogen pressure. The reaction was rapid, taking up an estimated 0.3 mol of hydrogen in half an hour. The platinum dioxide was removed from the solution by filtering on Hyflo Super Cel and the filtrate was treated with activated charcoal and filtered on glass fiber paper to give a pinkish solution. The ethanol was evaporated on a rotary evaporator to give a brownish oil. The product was distilled through a 10.16 cm (4") long vigreaux column giving a single fraction (88—92°C/0.13 mm) of a clear, viscous liquid. Distilled yield of tricyclo[5.2.1.$0^{2,6}$]decan-8-oxyethyl alcohol (*5*) was 90% by weight of the theoretical yield. The nmr spectrum was consistent with the expected product.

The saturated alcohol *5* was used in reactions analogous to Examples 1, 2, and 3 to prepare the saturated product *6*. In this instance, the procedure of Examples 1 and 2 were combined in a single step.

Example 5

Sodium Di-(tricyclo[5.2.1.$0^{2,6}$] dec-3-en-(8 or 9)-yl sulfosuccinate (*9*), the analog of *4* without the ethyleneglycol bridge, was prepared from the formate by transesterification as described stepwise in Steps A, B and C below:

*Step A:* Preparation of Tricyclo[5.2.1.$0^{2,6}$]dec-3-en-(8 or 9)-yl formate (*7*).

A 3 litre, 4-necked, round-bottom flask fitted with a thermometer, stirrer, condenser, addition funnel and nitrogen inlet was charged with 613.8 g (11.7 mol) formic acid (Mallinkrodt, purity 88% by weight) and 10.2 g of 98% by weight methane sulfonic acid. The mixture was heated under nitrogen to 100—103°C and then 1251 g (9 mol) of 95% by weight dicyclopentadiene was added over about 1 h. The reaction mixture was maintained at about 103°C for about 4.5 h, and was sampled hourly and analyzed by GLPC (gas/liquid partition chromatography, 0.5 m × 0.32 cm ($\frac{1}{8}$"), 5% OV 101 on Chromosorb HPG, 70°C (1 min) programmed 10°C/min to 200°C (10 min)). The dicyclopentadiene (retention time ($t_r$) = 2.53 min) decreased to 0% as the product peaks ($t_r$ = 5.61 and 6.54 min) increased to 4.8 and 82 area% respectively.

The reaction mixture was neutralized with 11 g of sodium carbonate. The flask was then equipped with a 10-plate Oldershaw column and distillation head and the reaction mixture was vacuum distilled. The forerun (48°C/78 mm — 20°C/15 mm) was hazy (water, formic acid). The product was distilled mainly at 121—122°C/16—17 mm. Total distilled yield of *7* was 1416.6 g (88% of theoretical yield).

*Step B:* Preparation of Di(tricyclo[5.2.1.$0^{2,6}$]dec-3-en-(8 or 9)-yl Maleate (*8*).

A 3 litre, 4-neck round-bottom flask, fitted with a thermometer, 10-plate Oldershaw column, variable rate take-off head, and air inlet/sparge, was charged with 1527.5 g of the formate *7*, 504 g of methyl maleate, 600 g of toluene, 0.67 g hydroquinone and 9.54 g Tyzor TPT (tetraisopropoxy titanate). Upon adding the Tyzor TPT the solution turned dark brown/red. The take-off head was set to take-off material boiling below 35°C and the reaction was heated. The reaction mixture refluxed with a pot temperature about 126°C and a total of 360.8 g of distillate boiling below 35°C was collected during 9 h reflux. The reaction mixture was heated to 152°C for an additional 4 h.

10 ml of water was added to the reaction mixture to hydrolyze the catalyst. The Oldershaw column was removed and the reaction mixture was vacuum distilled to remove excess formate *7* and toluene. A total of 267 g of unreacted *7* was distilled at 99°C/7 mm to about 91°C/1.5 mm.

The residue in the flask was dissolved in toluene to lower its viscosity and "filtered" on a bed of silica gel in a sintered glass funnel. A black sludge and much of the color was strongly adsorbed on the silica gel to yield a nearly colorless product. The product was concentrated by evaporating the excess toluene on a rotary evaporator (100°C). The product (1252.5 g) was analyzed by nmr and GLPC. It was estimated that the purity of the product (i.e. compound *8*) was 91% by weight — the major impurities were some residual toluene, *7*, and the mixed ester.

*Step C:* Sodium Di(tricyclo[5.2.1.$0^{2,6}$]dec-3-en-((8 or 9)-yl sulfosuccinate (*9*).

The addition of sodium metabisulfite to *8* is completely analogous to Example 3. The product was prepared at 68.2% by weight total solids. The solvent contained 31% by weight isopropanol to give a lower viscosity liquid.

Example 6

Polymerization Efficiency of the Polymerizable Surfactants

In order to evaluate the efficiency of the various surfactants in latex preparation, a simple screening procedure was developed. The surfactants were used to prepare a low solids polystyrene latex and the rate of polymerization was measured. Upon completion, the particle size was determined by Nanosizer. The rate should follow classical kinetics and the rate (%/min) should be linear in the so-called stage two. The rate and particle size should be indicative of the efficiency of the surfactant in generating particles.

General Procedure

A 1 litre, 4-necked, round-bottom flask fitted with stirrer, thermometer, condenser and nitrogen inlet was charged with 350 g double deionized water, 100.0 ml (90 g) styrene monomer, and the desired amount of the particular surfactant. The control was 0.9 g of siponate DS—10 (1% based on styrene) and other surfactants were employed on an equivalent basis to this control. The reaction mixture was stirred and heated to 50°C under the nitrogen flow. About 20 min after reaching temperature, a solution of 0.45 g potassium persulfate in 50 g water was added. The reaction was held at 50°C and sampled for analysis of the residual styrene by GLPC. Time-% styrene plots were evaluated to find the linear stage two region and the rate was computed as % styrene reacting per min. Upon completion, the reaction was filtered and particle size was measured on a Nanosizer. Conversion was measured by solids determination. Data are found in Table 1.

TABLE 1

Particle Size and Rate of Reaction in Styrene Screening Runs

The surfactants are described in terms of the two groups attached to the sulfosuccinate moiety (except for the Siponate DS—10 control). For the purpose of this Table the tricyclo [5.2.1.$0^{2,6}$]dec-3-en-(8 or 9) oxyethyl alcohol moiety is called DCPOE.

| Surfactant | % Wt. | Rate (%/min) | Particle Size (nm) | Conversion |
|---|---|---|---|---|
| DS—10 | 1.0 | 0.445 | 99 | 96.3 |
| 2-Ethylhexyl 2-Ethylhexyl | 1.3 | 0.481 | 103 | 97.1 |
| DCPOE Butyl | 1.28 | 0.328 | 128 | 93.5 |
| DCPOE Octyl | 1.46 | 0.457 | 98 | 92.4 |
| DCPOE DCPOE | 1.64 | 0.676 | 86 | 96.3 |
| DCPOE Butyl Carbitol | 1.52 | 0.325 | 134 | 94.9 |
| DCPOE Carbitol | 1.54 | * | 144 | 93.7 |

*Reaction experienced long induction period, then increase in rate. The reaction, as a result, was not followed long enough to establish the stage two polymerization.

Conversions do not reflex anything about the surfactant but merely indicate the point reached when particle size and solids analysis was done.

The rates increase and particle size decreases as the surfactant becomes more hydrophobic. The DCPOE/Octyl and DCPOE/DCPOE surfactants are as good or better than di(2-ethylhexyl)sulfosuccinate or Siponate DS—10 at controlling particle size and rate of reaction in this recipe.

Example 7

Grafting Efficiency of the Tricyclo[5.2.1.$0^{2,6}$]dec-3-enyl Containing Surfactants

The two surfactants *4* and *6* were found to hydrolyze in base and when injected into a gas chromatograph (gc) in the presence of water. Therefore, any of the alcohol moiety not grafted to polymer backbone could be easily quantified. To determine the efficiency of attaching the surfactants to a latex, a 65 methyl methacrylate/30 butyl acrylate/5 methacrylic acid latex was prepared by conventional methods using each of the two surfactants *4* and *6* at 1% by weight based on total potential polymer solids. To samples of each of these latexes was added 1% by weight of the same surfactant thereby resulting in the following four samples, (a) and (c) being as referred to as unspiked samples and (b) and (d) being referred to as spiked samples:—

(a) latex prepared using surfactant *4*;
(b) latex prepared using surfactant *4* with subsequent addition of 1% of same surfactant;
(c) latex prepared using surfactant *6*;
(d) latex prepared using surfactant *6* with subsequent addition of 1% of same surfactant.

These four samples were then hydrolyzed in basic acetone and analyzed for the resulting alcohol *1* and *5*. Undecyl alcohol was used as an internal standard. With the unsaturated surfactant *4*, 39% by weight of the possible alcohol was observed in the unspiked sample plus almost all the additional surfactant spiked into the latex was found in that sample. In the analogous latex made with the saturated surfactant *6*, about 90% of the possible alcohol was found in both the unspiked and spiked latex. These should represent a minimum since only one of the alcohol moieties of the disubstituted surfactants needs to be grafted or polymerized to attach the surfactant, and this leaves the other one free to hydrolyze.

Example 8

Foaming Behaviour of Latexes Made with the Polymerizable Surfactants

A series of latexes made with various surfactants, all having approximately the same particle size and composition, were diluted to 15% solids and evaluated for foam height and rate of foam break in a Ross-Miles apparatus. The conventional surfactant (Alipal CO—436) and three sulfosuccinates: Aerosol OT (di-2-ethylhexyl), Trem LF—40 (allyl, dodecyl), and surfactant 4 were compared. The sulfosuccinates were the best in terms of rapidity of foam break and the two potentially polymerizable ones were better than Aerosol OT. Surfactant *4* was the best of the series.

## Claims

1. A compound of the formula:—

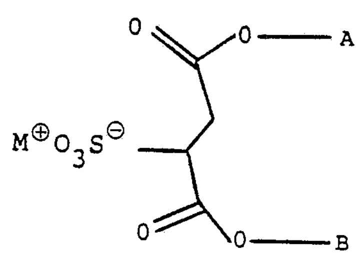

wherein M is a cation which is alkali metal, ammonium, amino or hydrogen; and each of A and B is a group which, together with the carboxyl group to which it is attached, forms an ester, at least one of A and B being a group having the formula:—

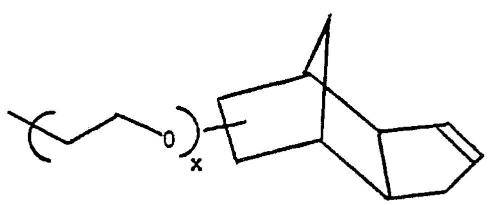

wherein x is from 0 to 5.

2. A compound as claimed in Claim 1, wherein one of A and B is an alkyl group of 2 to 8 carbon atoms, preferably octyl.

3. A compound as claimed in Claim 1, wherein one of A and B is a group having the formula:—

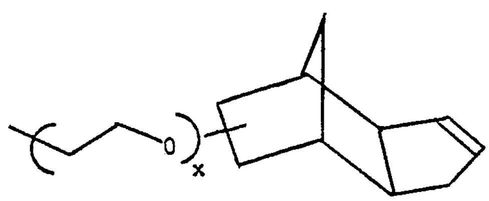

wherein x is from 0 to 5, and the other of A and B is a group having the formula:—

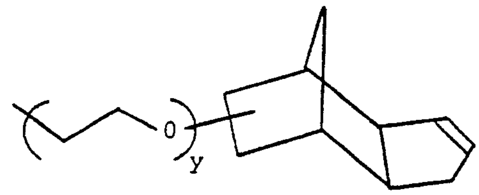

wherein y is from 0 to 5.

4. A compound as claimed in Claim 3, wherein x and y are each 0.

5. A compound as claimed in any of Claims 1 to 5, wherein M is sodium or ammonium.

6. A process, which comprises polymerizing, preferably emulsion polymerizing, one or more monomers, preferably comprising one or more ethylenically unsaturated monomers, to form polymer and wherein there is used, as graftable and/or copolymerizable surfactant, one or more compound as claimed in any of Claims 1 to 5.

7. A process as claimed in Claim 6, wherein said monomer(s) is/are one or more of acrylates, methacrylates, styrene, substituted styrenes, acrylonitrile, methacrylonitrile, vinyl alkanoates and butadienes.

8. A process as claimed in Claim 6 or 7, wherein said surfactant is present in an amount of 0.1 to 5%, preferably 1 to 3%, by weight based on the weight of monomer(s).

9. A process as claimed in any of Claims 6 to 8, wherein emulsion polymer is formed in which the particles have a weight average diameter of 40 to 200, e.g. 50 to 110, nanometers.

10. A process for preparing a compound as claimed in Claim 1, which comprises reacting dicyclopentadiene with one or more of ethylene glycol, diethylene glycol, triethylene glycol, tetraethylene glycol and pentaethylene glycol, and reacting the resulting [5.2.1.0.$^{2,6}$]dec-3-en-(8 or 9 oxyethyl) alcohol, wherein n is from 0 to 5, and optionally another alcohol, with maleic anhydride and bisulfite ion.

**Patentansprüche**

1. Verbindung der Formel

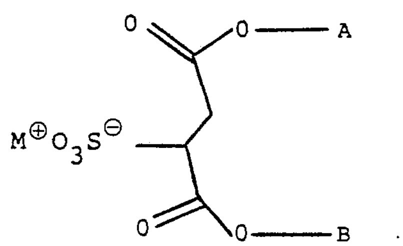

worin M ein Kation ist, das ein Alkalimetall, Ammonium, Amino oder Wasserstoff ist, und jede der Gruppen A und B eine Gruppe ist, die zusammen mit der Carboxylgruppe, mit der sie verknüpft ist, einen Ester bildet, wobei wenigstens eine der Gruppen A und B eine Gruppe der Formel

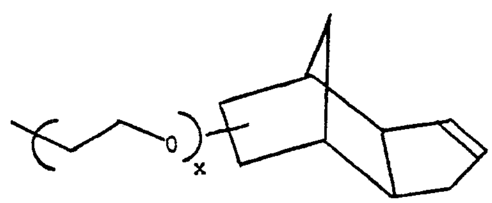

ist, worin x 0 bis 5 ist.

2. Verbindung nach Anspruch 1, worin eine der Gruppen A und B eine Alkylgruppe mit 2 bis 8 Kohlenstoffatomen, vorzugsweise Octyl, ist.

3. Verbindung nach Anspruch 1, worin eine der Gruppen A und B eine Gruppe der Formel

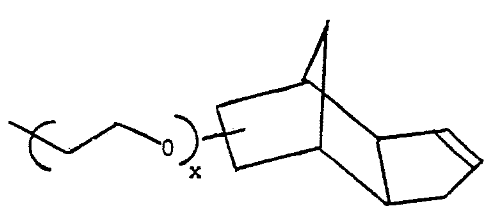

ist, worin x 0 bis 5 ist, und die andere der Gruppen A und B eine Gruppe der Formel

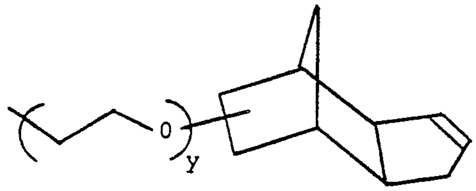

ist, worin y 0 bis 5 ist.

4. Verbindung nach Anspruch 3, worin x und y jeweils 0 sind.

5. Verbindung nach einem der Ansprüche 1 bis 5, worin M Natrium oder Ammonium ist.

6. Verfahren, welches darin besteht, vorzugsweise ein Monomeres oder mehrere Monomere, das bzw. die ein ethylenisch ungesättigtes Monomeres oder mehrere ethylenisch ungesättigte Monomere aufweist bzw. aufweisen, unter Bildung eines Polymeren in Emulsion zu polymerisieren, wobei als pfropfbares und/ oder copolymerisierbares grenzflächenaktives Mittel eine oder mehrere Verbindung(en) gemäß einem der Ansprüche 1 bis 5 verwendet wird (werden).

7. Verfahren nach Anspruch 6, worin das Monomere (die Monomeren) aus einer oder mehreren der folgenden Verbindungen besteht (bestehen): Acrylaten, Methacrylaten, Styrol, substituierten Styrolen, Acrylnitril, Methacrylnitril, Vinylalkanoaten und Butadienen.

8. Verfahren nach Anspruch 6 oder 7, worin das grenzflächenaktive Mittel in einer Menge von 0,1 bis 5 Gew.-%, vorzugsweise 1 bis 3 Gew.-%, bezogen auf das Gewicht des Monomeren bzw. der Monomeren, vorliegt.

9. Verfahren nach einem der Ansprüche 6 bis 8, worin ein Emulsionspolymeres gebildet wird, in welchem die Teilchen einen Gewichtsmitteldurchmesser von 40 bis 200, beispielsweise 50 bis 110, nm besitzen.

10. Verfahren zur Herstellung einer Verbindung gemäß Anspruch 1, welches darin besteht, Dicyclopentadien mit einer oder mehreren der Verbindungen Ethylenglykol, Diethylenglykol, Triethylenglykol, Tetraethylenglykol und Pentaethylenglykol umzusetzen und den erhaltenen $[5.2.1.0^{2,6}]$dec-3-en-(8- oder 9-oxyethyl)-Alkohol, worin n 0 bis 5 ist, und gegebenenfalls einen anderen Alkohol mit Maleinsäureanhydrid und Bisulfition umzusetzen.

**Revendications**

1. Un composé de formule:

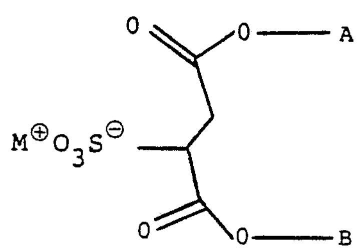

dans laquelle M est un cation qui est un métal alcalin, un ammonium, un amino ou un hydrogène; et chacun de A et B est un groupe qui, avec le groupe carboxyle auquel il est fixé, forme un ester, au moins un de A et B étant un groupe de formule:

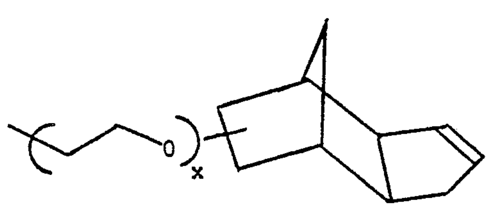

dans laquelle x vaut de 0 à 5.

2. Un composé comme revendiqué dans la revendication 1, où un de A et B est un groupe alcoyle de 2 à 8 atomes de carbone, de préférence octyle.

3. Un composé comme revendiqué dans la revendication 1, où un de A et B est un groupe de formule:

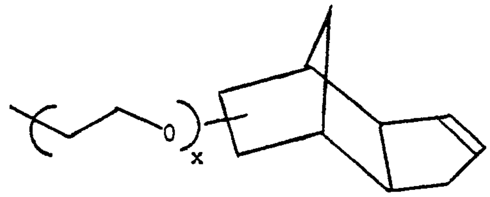

où x vaut de 0 à 5 et l'autre de A et B est un groupe de formule:

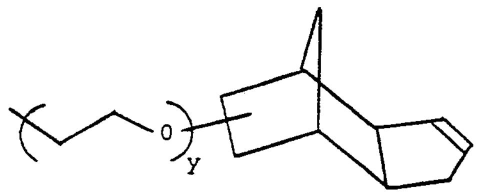

où y vaut de 0 à 5.

4. Un composé comme revendiqué dans la revendication 3, où x et y sont chacun 0.

5. Un composé comme revendiqué dans l'une quelconque des revendications 1 à 5, où M est le sodium ou l'ammonium.

6. Un procédé qui comprend la polymérisation, de préférence la polymérisation en émulsion, d'un ou plusieurs monomères, comprenant de préférence un ou plusieurs monomères à insaturation éthylénique, pour former un polymère et dans lequel on utilise comme agent tensio-actif greffable et/ou copolymérisable un ou plusieurs composés comme revendiqué dans l'une quelconque des revendications 1 à 5.

7. Un procédé comme revendiqué dans la revendication 6, où ledit (lesdits) monomère(s) est/sont un ou plusieurs des acrylates, des méthacrylates, du styrène, des styrènes substitués, de l'acrylonitrile, du méthacrylonitrile, des alcanoates de vinyle et des butadiènes.

8. Un procédé comme revendiqué dans la revendication 6 ou 7, où ledit agent tensio-actif est présent en une quantité de 0,1 à 5%, de préférence de 1 à 3% en poids par rapport au poids du (des) monomère(s).

9. Un procédé comme revendiqué dans l'une quelconque des revendications 6 à 8, où il se forme un polymère d'émulsion dont les particules ont une moyenne pondérale du diamètre de 40 à 200, par exemple de 50 à 110 nanomètres.

10. Un procédé pour préparer un composé comme revendiqué dans la revendication 1, qui comprend la réaction du dicyclopentadiène avec un ou plusieurs de l'éthylèneglycol, du diéthylèneglycol, du triéthylèneglycol, du tétraéthylèneglycol et du pentaéthylèneglycol, et la réaction de l'alcool $[5.2.1.0^{2,6}]$déc-3-ène-(8 ou 9-oxyéthylique) dans lequel n vaut de 0 à 5 et, facultativement, un autre alcool avec l'anhydride maléique et l'ion bisulfite.